# FASCICULE DE BREVET EUROPEEN

(11) **EP 1 407 247 B1**
(45) Date de publication et mention de la délivrance du brevet: **11.02.2009**
(21) Numéro de dépôt: 02794803.3
(22) Date de dépôt: 14.06.2002
(51) Int. Cl.: G01N 17/00, G01N 33/44

(54) **PERFECTIONNEMENTS AUX SYSTEMES DE TESTS DE VIEILLISSEMENT ARTIFICIEL ACCELERE**
VERBESSERUNGEN AN BESCHLEUNIGTEN KÜNSTLICHEN VERWITTERUNGSTESTSYSTEMEN
IMPROVEMENTS TO ACCELERATED ARTIFICIAL WEATHERING TEST SYSTEMS

(30) Priorité: 14.06.2001 FR 0108234; 14.06.2002 FR 0207772
(43) Date de publication de la demande: 14.04.2004
(73) Titulaire: Beraud, Michel P.P., 83150 Bandol (FR)
(72) Inventeur: Beraud, Michel P.P., 83150 Bandol (FR)
(74) Mandataire: Bentz, Jean-Paul
(86) Numéro de dépôt international: PCT/FR2002/002073
(87) Numéro de publication internationale: WO 2003/016876

(56) Documents cités:
- EP-A- 0 289 436
- EP-A- 0 320 209
- EP-A- 0 403 281
- WO-A-01/44787
- DE-A- 3 243 722
- DE-A- 19 945 917
- FR-A- 2 802 301
- US-A- 4 760 748
- US-A- 4 770 542
- US-A- 5 206 518
- US-A- 5 646 358
- PATENT ABSTRACTS OF JAPAN vol. 013, no. 017 (P-813), 17 janvier 1989 (1989-01-17) & JP 63 222240 A (KUBOTA LTD), 16 septembre 1988 (1988-09-16)

## Description

### ARRIERE-PLAN DE L'INVENTION

### 1. Domaine Technique

La présente invention concerne le domaine des dispositifs et appareils à source de rayonnements électromagnétiques pour tester le vieillissement accéléré d'échantillons sous l'effet de la lumière, ainsi que d'autres conditions atmosphériques, comme la température, l'humidité ou les précipitations d'eau... Les essais portent en particulier sur la tenue d'échantillons en matériaux polymères qui sont essentiellement sensibles aux rayonnements ultraviolets.

### 2. Etat de la Technique Antérieure

De façon connue, le vieillissement naturel des matériaux à la lumière solaire est simulé en exposant des échantillons à une source de rayonnements électromagnétiques dont la répartition spectrale doit être soigneusement choisie pour obtenir un vieillissement accéléré corrélable à celui constaté dans les conditions naturelles.

On connaît divers appareillages d'exposition à des sources lumineuses de laboratoire. La nature des sources de rayonnement de lumière a une influence prépondérante sur les conditions d'essai et détermine entièrement le fonctionnement des dispositifs.

La norme ISO 4892 qui régit les 'Méthodes d'Exposition à des sources lumineuses de laboratoire' pour les 'Plastiques', distingue ainsi trois catégories de sources de rayonnement et contient, outre le Guide Général en Partie 1 (ISO 4892-1), trois annexes qui visent respectivement :
- Partie 2 : Sources à arc au xénon (ISO 4892-2)
- Partie 3 : Lampes fluorescentes U.V. (ISO 4892-3)
- Partie 4 : Lampes à arc carbone à feu nu (ISO 4892-4).

Dans la catégorie des lampes à arc de carbone, historiquement la plus ancienne, la source lumineuse est formée de plusieurs paires d'électrodes au carbone, typiquement 2 à 4 paires d'électrodes, composées d'un mélange de sels métalliques de terres rares avec une métallisation de surface, les électrodes opposées étant disposées axialement dans le prolongement l'une de l'autre avec un système pour maintenir leur écartement constant au fur et à mesure qu'elles se consomment.

L'arc électrique entre les électrodes de carbone engendre un rayonnement qui contient à la fois des rayons ultraviolets, de la lumière visible et infrarouge, tout en dégageant une très grosse quantité de chaleur.

De ce fait, les échantillons ne peuvent pas être exposés directement à la flamme nue des lampes à arc de carbone et la lumière doit être filtrée, différents filtres et éléments de protection étant préconisés.

La norme ISO 4892-4 préconise des filtres en verre résistant à la chaleur, ce qui convient pour simuler une exposition au soleil à travers un verre de vitre épais, en Pyrex, Corex, etc. ; ces filtres en verre doivent avoir une composition et une épaisseur donnant un spectre de transmission spécifique.

Dans cette catégorie d'appareillages dotés de lampes à arc au carbone, on connaît une série de documents relatifs à des dispositifs de tests accélérés de stabilité à la lumière et aux agents climatiques, produits par la Société japonaise SUGA Test Instrument Co..

Le document US-4, 760, 748 au nom de SUGA, publié en 1988 et la lignée de documents antérieurs US-B-4,704,903 et US-B-3,983,742 décrivent tous des dispositifs constitués par un large châssis cylindrique plein qui tourne autour d'une source lumineuse constituée d'un cylindre cheminée tubulaire vertical contenant les paires d'électrodes disposées longitudinalement. Les éprouvettes d'échantillons polymères sont disposées dans des ouvertures aménagées dans les parois pleines du châssis cylindrique.

Le cylindre cheminée (en anglais "furnel" Il ou tuyau de cheminée de poêle) est percé de fenêtres dans lesquelles sont disposés les filtres.

Le cylindre cheminée ou carter cylindrique qui porte les éléments de filtre en verre, s'interpose ainsi entre le brûleur à arc d'électrodes au carbone et les échantillons qui sont logés dans les ouvertures du châssis tubulaire porte-échantillons pour empêcher que le fort rayonnement infrarouge et l'important dégagement d'air chaud de la flamme des électrodes de carbone atteigne la surface des échantillons.

Le châssis porte-échantillons tourne autour de l'axe central du carter de brûleur à arc de carbone, qui est vertical ou légèrement incliné selon les préconisations ISO 4892.4 (point4.2). Le châssis porte-échantillons a un encombrement hors tout important, avec des dimensions métriques, typiquement 96 centimètres.

Du fait de l'important dégagement de chaleur, les dispositifs à lampe à arc de carbone ont un double système de refroidissement complexe pour, d'une part, refroidir le brûleur et, d'autre part, tempérer les échantillons.

On connaît des dispositifs comprenant une rampe fixe de tubes à arc basse pression qui fournissent des rayonnements ultraviolets. Cette rampe de tubes parallèles est disposée en face des échantillons à tester.

L'inconvénient du rayonnement des tubes à arc basse pression est d'avoir une faible émissivité et un spectre ultraviolet très différent du spectre solaire, ce qui obère les essais.

Autre inconvénient, ces dispositifs ne permettent pas d'obtenir une exposition homogène des échantillons, si bien que les résultats d'essais ont une mauvaise reproductibilité.

On connaît des dispositifs utilisant des lampes à arc au Xénon, qui ont l'intérêt de présenter un spectre très proche du spectre solaire, lorsqu'elles sont filtrées convenablement.

Selon l'état des développements récents de la technique, on peut maintenant adopter de façon équivalente des lampes à vapeur de mercure moyenne pression filtrées, dont le spectre est très riche eh rayonnements ultraviolets, et présente un bon équilibre entre les ultraviolets longs, de type U.V.A., et les ultraviolets courts, de type U.V.B., ce qui permet de bien reproduire le vieillissement dû à la lumière solaire.
On connaît des appareils d'essais de vieillissement dans lesquels les échantillons sont montés sur un porte-échantillons cylindrique vertical rotatif autour de lampes à arc au Xénon de forme tubulaire disposées verticalement.

La rotation des échantillons autour des lampes au Xénon, permet de régulariser l'exposition au rayonnement.

- Cependant ces lampes présentent un fort dégagement thermique et il faut prévoir une circulation d'air en circuit ouvert ou en circuit fermé pour maintenir les échantillons à une température contrôlée.

Ces appareils ont l'inconvénient d'exposer les échantillons à des température hétérogènes .

En effet, le circuit de ventilation comporte une zone d'aspiration d'air au-dessus du porte-échantillons cylindrique qui provoque un flux d'air vertical comme dans une cheminée. Le flux d'air se réchauffe alors en progressant verticalement le long des lampes et des échantillons, si bien que les échantillons disposés en haut du porte-échantillons se trouvent nécessairement à une température supérieure à celle des échantillons disposés en bas. Des échantillons identiques situés à des hauteurs différentes sont donc soumis à des températures différentes, ce qui affecte encore ' la reproductibilité des essais. Dans la pratique certains utilisateurs sont contraints d'interrompre les essais pour intervertir les échantillons en cours de test, entre le haut et le bas du porte-échantillons.

On connaît aussi des dispositifs comportant une chambre d'essais parallélépipédique avec des lampes au mercure disposées verticalement aux quatre coins de la chambre, autour d'un petit cylindre rotatif porte-échantillons disposé au centre de la chambre. Les lampes et la face externe des échantillons sont refroidies par une circulation d'air ventilé avec des arrivées d'air ouvertes dans les parois latérales de la chambre et une sortie d'air coiffant tout le cylindre porte-échantillons au plafond de la chambre.

Un inconvénient de ce dispositif est de présenter un encombrement excessif par rapport à la faible surface d'échantillons en test.

Ce dispositif a aussi l'inconvénient de présenter d'importantes pertes d'énergie lumineuse, puisque plus de 75% de la lumière ne se dirige pas directement vers le porte-échantillons.

Consécutivement, ce dispositif présente un mauvais rendement lorsqu'on rapporte l'amortissement du matériel ajouté aux dépenses énergétiques, à la surface d'échantillons en test, ce qui prétérite le coût des essais.

Il existe encore des appareils comportant une chambre d'essais rectangulaire dans laquelle est montée une cage porte-échantillons cylindrique, montée à rotation sur un arbre d'entraînement plein. Les échantillons sont longés par un flux d'air laminaire se propageant verticalement entre des ouvertures percées dans l'axe des génératrices du cylindre au plancher et au plafond de la chambre, dans le but d'isoler les échantillons du flux d'air chaud issu de la lampe et d'homogénéiser les températures d'essais.

Malgré le flux d'air laminaire, cet appareil a toujours l'inconvénient d'exposer les échantillons à des températures différentes, le flux d'air laminaire se réchauffant encore au contact des échantillons exposés au fort rayonnement lumineux. Dans la pratique, les échantillons disposés en haut de la cage sont ainsi exposés à une température supérieure de plusieurs degrés à celle des échantillons disposés en bas de la cage.

Le brevet EP-0 320 209 décrit ainsi une armoire de test atmosphérique comprenant un rack ou panier porte-échantillons rotatif autour d'un tube lumineux au Xénon, disposé verticalement. Les échantillons sont refroidis par un flux d'air laminaire qui longe verticalement les parois internes des échantillons disposés le long des flancs droits du rack. Par économise, le flux d'air a un débit limité.

Cette armoire de test à flux d'air laminaire vertical à faible débit a l'inconvénient de ne pas apporter un refroidissement efficace des échantillons et d'induire des différences de température entre les échantillons disposés en bas et ceux disposés en haut du rack.

Le brevet US-4,760,748 décrit un autre appareil de test de vieillissement accéléré comprenant également un châssis cylindrique porte-échantillons monté en rotation autour d'un axe vertical et de tubes lumineux, avec un flux d'air laminaire ascendant refroidissant la face interne des échantillons. Le châssis cylindrique comporte des parois pleines percées de deux rangées d'ouvertures pour d'une part, disposer les échantillons, d'autre part former des orifices d'aspiration d'un flux d'air secondaire.

En fait, pour le refroidissement, l'appareil comporte un système de circulation d'air à deux flux, avec une colonne d'air principale ascendante, qui monte dans le cylindre porte-échantillons en longeant la face interne des échantillons et un flux d'air secondaire périphérique venant frapper la face externe des échantillons. Le flux d'air secondaire d'appoint provient d'une source d'air refroidi. Le flux d'air secondaire est créé par un effet d'aspiration provoqué au niveau des orifices du cylindre par l'ascension de la colonne d'air principale. Les orifices ont une obturation variable pour doser le flux d'air secondaire périphérique par rapport au flux d'air principal ascendant.

L'inconvénient de cet appareil est la complexité et l'encombrement particulièrement élevé de son système de circulation d'air à double flux.

Autre inconvénient, l'appareil présente un espace de test et une capacité en nombre d'échantillons bien restreints par rapport à l'encombrement volumineux de l'armoire avec son appareillage pour l'évacuation d'air en partie haute, la soufflerie et l'entraînement en rotation en partie basse, ainsi que le système de circulation d'air périphérique et la source d'air froid latérale.

Ce système de refroidissement à deux flux d'air a encore l'inconvénient d'induire des différences de température conséquentes entre la face interne irradiée et la face externe refroidie des échantillons.

On connaît par le document allemand DE-A-32 43 722, un dispositif bien différent d'essais de résistance à la lumière et aux intempéries comprenant une grande enceinte ventilée dans laquelle est disposé un ensemble horizontal comprenant une couronne de tubes lumineux insérées entre deux conduites d'air concentriques horizontales et un tambour porte-échantillons monté en rotation autour des conduites horizontales. Les deux conduites sont reliées à un ventilateur et communiquent entre elles ainsi qu'avec l'extérieur de l'enceinte de sorte que l'air extérieur est insufflé dans la première conduite puis retourne et s'insère dans l'espace entre la première et la seconde conduite pour longer les tubes lumineux en les refroidissant en revenant vers l'extérieur.

Quant aux échantillons disposés sur le tambour externe, ils sont refroidis séparément par un autre flux d'air vertical qui circule dans un circuit doté d'un autre ventilateur et d'un échangeur de refroidissement ainsi qu'éventuellement de moyens de chauffage.

Ce dispositif comporte donc deux systèmes de circulation d'air refroidissant d'une part les échantillons, d'autre part les tubes lumineux.

Dans ce dispositif, les tubes lumineux ainsi qu'un système d'aspersion sont disposés dans des conduites qui les séparent des échantillons, ce qui a l'inconvénient de nuire particulièrement à l'exposition lumineuse des échantillons et à l'efficacité du système.

Ce dispositif a encore l'inconvénient que le flux d'air vertical qui traverse le tambour porte-échantillons n'apporte pas un refroidissement efficace aux échantillons.

L'objet de la présente invention est de réaliser un dispositif d'exposition à des rayonnements pour tester le vieillissement d'échantillons permettant de soumettre les échantillons à une température et à une irradiation uniformes, sans les inconvénients précités.

L'invention a en particulier pour objectif d'assurer une ventilation efficace des échantillons lors des tests effectués avec le dispositif.

Un autre objectif de l'invention est d'obtenir un dispositif présentant un nombre de lampe et un encombrement réduits.

Un autre objectif parallèle de l'invention est d'obtenir un dispositif de conception simple et de rendement lumineux élevé rapporté à la surface d'échantillons exposés de façon à réduire le coût des essais.

Enfin, l'invention a aussi pour objectif de faciliter la manipulation des échantillons préliminaire aux tests de vieillissement.

### EXPOSE SOMMAIRE DE L'INVENTION

Succinctement ces objectifs sont atteints selon l'invention, en réalisant un dispositif comportant une cage porte-échantillons rotative dont l'axe de rotation est de préférence disposé horizontalement, de sorte que les échantillons se trouvent cycliquement dans la partie supérieure, puis dans la partie inférieure de l'enceinte, ce qui permet de s'abstraire des différences de températures inhérentes, et plus essentiellement en prévoyant que le dispositif présente un flux d'air arrivant directement sur la lampe, insufflé par un orifice axial, puis divergeant autour de la lampe et s'évacuant dans le prolongement radial de la cage porte-échantillons, à travers des ouvertures de sorties d'air disposées à la périphérie de la cage, si bien que le flux d'air s'échauffe au contact de la lampe avant d'envelopper les échantillons en élevant puis en régularisant leur température. De préférence, le système de circulation d'air forme un flux d'air tourbillonnaire autour de l'axe de la cage.

Dans le dispositif le flux d'air entre au centre de la cage parallèlement à l'axe, en selon l'invention, ayant essentiellement une composante axiale dans la partie centrale de la cage, puis s'évacue à la périphérie de la cage en arrivant transversalement à l'axe et en ayant essentiellement des composantes tangentielles et/ou radiales à la périphérie de la cage, ce qui assure avantageusement une symétrie et une régularité du flux d'air.

L'invention est réalisée avec un dispositif d'exposition à des rayonnements photoniques pour des essais de vieillissement artificiel accéléré d'échantillons, comprenant :
- une enceinte,
- une cage porte-échantillons rotative autour d'un axe destiné à être sensiblement horizontal en fonctionnement,
- des moyens pour positionner fixement dans la partie centrale de la cage et pour alimenter électriquement, au moins une lampe à arc de faible puissance de l'ordre de plusieurs dizaines à un milliers de Watts, notamment une lampe à vapeur de mercure ou à vapeur de composé mercuriel, et
- un système de circulation d'air comportant un orifice de passage d'air disposé dans le prolongement axial de la partie centrale de la cage et plusieurs ouvertures fixes de passage d'air disposées dans le prolongement radial de la périphérie de la cage, la circulation d'air étant destinée à maintenir les échantillons dans l'enceinte à une température homogène s'élevant à plusieurs dizaines de degrés Celsius, l'amélioration disposant que des moyens de ventilation insufflent l'air dans l'enceinte à travers l'orifice qui forme un orifice d'arrivée d'air dirigé sensiblement parallèlement à l'axe horizontal, la lampe étant disposée dans le prolongement axial immédiat de l'orifice d'arrivée d'air, de façon à générer un flux d'air arrivant directement sur la lampe et sensiblement parallèle à l'axe dans la partie centrale de la cage, le flux d'air divergeant autour de la lampe et s'évacuant sensiblement perpendiculaire à l'axe par les ouvertures fixes qui forment des ouvertures de sortie d'air, à la périphérie de la cage, si bien que le flux d'air s'échauffe au contact de la lampe, avant d'envelopper les échantillons en élevant puis en régularisant leur température.

Selon un autre perfectionnement essentiel, il est prévu que le dispositif comporte une cuve disposée en partie inférieure de l'enceinte pour baigner la partie inférieure de la cage porte-échantillons rotative.

Cette disposition permet avantageusement d'alterner des phases d'exposition des échantillons à une immersion dans le liquide et une des phases d'émersion dans le flux d'air avec exposition au rayonnement U.V. de la lampe.

Ce perfectionnement avantageux permet d'envisager toute une série de protocoles d'essais avec différentes natures de liquides correspondant aux facteurs de dégradation et de multiples variantes d'alternance de phases d'immersion/émersion, et d'éclairage/obscurité.

Selon ce perfectionnement, le meilleur mode de réalisation de l'invention est alors obtenu avec une exposition à des rayonnements photoniques et à des agents climatiques pour réaliser des tests de vieillissement artificiel accéléré d'échantillons comprenant :
- une enceinte,
- une cage porte-échantillons rotative autour d'un axe (destiné à être disposé sensiblement horizontal en fonctionnement,
- des moyens pour positionner fixement, et pour alimenter électriquement, au moins une lampe à rayonnement électromagnétique, dans la partie centrale de la cage, selon l'axe horizontal,
- des moyens pour générer un flux d'air direct simple dans l'enceinte, le flux d'air se propageant directement et librement de la partie centrale interne à la partie périphérique externe de la cage et,
- une cuve disposée en partie inférieure du dispositif et destinée à contenir un volume de liquide de façon à baigner une portion inférieure de la cage porte-échantillons rotative, de sorte que les échantillons alternent cycliquement des phases d'immersion dans le liquide avec des phases d'émersion dans le flux d'air, afin de tester la tenue des échantillons au vieillissement sous l'action de l'immersion et des rayonnements photoniques (la résistance aux agents climatiques et la stabilité aux rayonnements photoniques des échantillons).

De préférence, la cuve est formée d'un bassin en forme de demi-lune fixé à la partie inférieure de la platine du dispositif.

Il est prévu des moyens d'alimentation qui maintiennent sensiblement constant le niveau de liquide dans la cuve.

Ainsi, avantageusement, la rotation de la cage porte-échantillons permet d'alterner des phases d'exposition des échantillons à une immersion dans l'eau avec des phases d'exposition des échantillons au flux d'air, tout en exposant les échantillons au rayonnement photonique de la lampe.

Préférentiellement, la source de rayonnements photoniques est une lampe à arc au mercure de faible puissance, de l'ordre de plusieurs dizaines à un millier de Watts.

Avantageusement, le flux d'air divergeant autour de la lampe comporte essentiellement une composante sensiblement parallèle à l'axe, dans la partie centrale de la cage, auprès de la lampe, alors que ledit flux d'air comporte essentiellement des composantes sensiblement perpendiculaires à l'axe, à la périphérie de la cage au niveau des échantillons.

Avantageusement, le flux d'air entre au centre de la cage parallèlement à l'axe, en ayant essentiellement une composante axiale dans la partie centrale de la cage, puis s'évacue à la périphérie de la cage en arrivant transversalement à l'axe et en ayant essentiellement des composantes tangentielles et/ou radiales. à la périphérie de la cage, ce qui assure une symétrie et une régularité du flux d'air.

Avantageusement, l'ensemble des moyens de positionnement et d'alimentation de la lampe, des moyens de ventilation et de circulation d'air et des moyens d'entraînement en rotation de la cage sont disposés dans une même partie axiale du dispositif.

Avantageusement, l'ensemble des moyens de positionnement et d'alimentation de la lampe, des moyens de ventilation et de circulation d'air et des moyens d'entraînement en rotation de la cage sont disposés en ligne suivant l'axe horizontal du dispositif.

De préférence, la cage est montée en rotation sur un moyeu évidé axialement, le flux d'air étant insufflé dans l'évidement axial du moyeu.

De préférence, le système de circulation d'air comporte des moyens de ventilation disposés dans la lumière d'évidement du moyeu, de façon à y insuffler l'air en direction de l'enceinte.

De préférence, le système de circulation d'air est en circuit ouvert, ce qui permet avantageusement d'aspirer directement l'air frais du laboratoire.
Alternativement, le système de circulation d'air peut fonctionner en circuit fermé.

De préférence, le système de circulation d'air comporte une série de plusieurs ouvertures fixes de sorties d'air disposées régulièrement à la périphérie de la cage avec une symétrie de révolution autour de l'axe, chaque ouverture fixe étant orientée dans une direction comprise entre la direction radiale et la direction tangentielle à la périphérie de la cage. Une conduite d'évacuation d'air est disposée dans le prolongement axial de la partie centrale de la cage, la conduite étant orientée dans une direction parallèle à l'axe.

Selon le mode de réalisation préféré de l'invention, l'enceinte cylindrique comporte des ouïes de sortie d'air disposées sensiblement à la périphérie de la cage, chaque ouïe de sortie d'air étant dirigée non-radialement. Il est prévu que chaque ouïe de sortie d'air a une profondeur, une ouverture et un angle d'inclinaison par rapport à la direction radiale, tels que le rayonnement de chaque lampe positionnée dans la partie centrale de la cage ne traverse pas l'ouïe et ne s'échappe pas directement hors de l'enceinte.

Selon le mode de réalisation préféré de l'invention, la cage comporte des éléments centraux et des éléments périphériques s'étendant dans la direction axiale et reliés par des éléments radiaux, les éléments centraux et périphériques s'étendant dans un seul demi-espace délimité par les éléments radiaux.

Selon le mode de réalisation préféré, la cage comporte des éléments circulaires de diamètres différents permettant de disposer des échantillons sous des angles d'exposition différents.

Selon une alternative avantageuse, les éléments centraux de la cage se développent en hélices autour de l'axe.

Selon le mode de réalisation préféré de l'invention, la cage est reliée au moyeu par un mécanisme d'entraînement débrayable, permettant une rotation libre de la cage.

De façon avantageuse, le mécanisme comporte des moyens de débrayage en rotation sous l'effet d'un couple axial limite entre la cage et le moyeu.

De façon avantageuse, le mécanisme comporte des moyens de débrayage ou de dé-solidarisation en translation sous l'effet d'un effort axial limite entre la cage et le moyeu.

Selon une alternative de réalisation, le dispositif comporte un réservoir d'eau disposé pour baigner une partie de la cage.

De préférence, un capteur de température est disposé fixement à l'intérieur de l'enceinte suivant un emplacement proche de la périphérie de la cage rotative porte-échantillons.

Il est prévu qu'un circuit d'asservissement contrôle la température en fonction d'une mesure et d'une consigne de température, en agissant sur l'alimentation électrique de la lampe et/ou sur la marche des moyens de ventilation. Il est prévu que le circuit d'alimentation électrique comporte un ballast pour réguler le courant d'alimentation d'une lampe à vapeur de Mercure moyenne pression.

Selon une variante, le système de circulation d'air comporte des moyens de chauffage du flux d'air.

Selon une autre variante, le système de circulation d'air comporte des moyens de réfrigération du flux d'air.

L'invention porte également sur un procédé de test de vieillissement d'échantillons ayant la particularité de mettre en oeuvre un tel dispositif d'exposition à des rayonnements.

Et surtout, tout en répondant à l'objet principal de la présente invention le perfectionnement du meilleur mode de réalisation de l'invention permet plus précisément de réaliser un procédé de test de vieillissement artificiel accéléré d'échantillons, dans lequel on expose des échantillons à des rayonnements photoniques et à des agents climatiques, caractérisé en ce qu'il met en oeuvre des étapes consistant à :
- disposer les échantillons (E, E1, E2, ..., E9) sur une cage (40) porte-échantillons rotative autour d'un axe sensiblement horizontal, (H-H)
- positionner fixement (15), et alimenter électriquement, au moins une lampe (10) à rayonnement photonique, dans la partie centrale (45) de la cage, selon l'axe de rotation horizontal (H-H),
- générer un flux d'air direct simple arrivant directement sur la lampe (10) en se propageant parallèlement sensiblement à l'axe horizontal (H-H) dans la partie centrale de la cage (45, 46), le flux d'air divergeant autour de la lampe et s'évacuant sensiblement perpendiculairement à l'axe horizontal (H-H) à la périphérie (41), de la cage porte-échantillons, si bien que le flux d'air s'échauffe au contact de la lampe, avant d'envelopper les échantillons, et
- baigner une portion inférieure de la cage porte-échantillons rotative dans un bain liquide, de façon à alterner cycliquement des phases d'immersion des échantillons dans le liquide avec des phases d'émersion des échantillons dans le flux d'air.

Ainsi, on baigne la portion inférieure de la cage dans une cuve apte à contenir un volume de liquide.

Il est prévu que le niveau de liquide baignant la partie inférieure de la cage est maintenu sensiblement constant.

Avantageusement, la rotation de la cage porte-échantillons permet d'alterner des phases d'exposition des échantillons à une immersion dans le liquide et des phases d'exposition des échantillons au flux d'air, tout en exposant les échantillons en rotation au rayonnement photonique de la lampe.

Selon un protocole d'essai, il est prévu d'effectuer des mesures de température du flux d'air en au moins un emplacement proche de la périphérie de la cage porte-échantillons rotative.

Par suite, il est possible de réguler la température de flux d'air par un asservissement de température agissant sur l'alimentation électrique de la lampe et/ou sur les moyens de génération du flux d'air.

Plus précisément, il est possible de maintenir une consigne de température de test de l'ordre de 35°Celsius à 100°Celsius.

Selon un autre protocole d'essai, il est possible de faire varier la température entre des valeurs extrêmes climatiques.

Alternativement, selon un autre protocole d'essais, il est possible de laisser varier la température librement.

De préférence, suivant le protocole d'essais, la durée de la phase d'immersion représente une proportion de l'ordre de quelques centièmes (est inférieure) à la moitié de la durée du cycle de rotation de la cage porte-échantillons.

Selon le protocole préféré, la durée de la phase d'immersion représente une proportion de l'ordre d'un dixième (de quelques centièmes) à un tiers de la durée du cycle de rotation de la cage porte-échantillons.

D'autre part, suivant le protocole d'essais, la vitesse de rotation de la cage porte-échantillons est inférieure à un tour par minute.

Selon le protocole préféré, la vitesse de rotation de la cage porte-échantillons est de l'ordre d'un dixième de tour à dix tours par heure.
Par ailleurs, plus fondamentalement et avantageusement, selon le protocole d'essai préféré, il est prévu de faire alterner des phases d'exposition au rayonnement photonique de la lampe et des phases de maintien dans l'obscurité.

Avantageusement, la durée de la phase de maintien dans l'obscurité représente une proportion de l'ordre d'un dixième à une moitié de la durée du cycle d'alternance des phases d'exposition au rayonnement et d'obscurité.

Selon un protocole d'essais, la phase de maintien dans l'obscurité coïncide, au moins partiellement, avec la phase d'immersion.

Maintenant, il est prévu selon le perfectionnement du procécé de test, divers protocoles d'essais avec des phases d'immersion dans lesquelles :
- de l'eau pure de potentiel Hydrogène égal à sept (pH=7,0).
- de l'eau contenant de l'oxygène dissous,
- éventuellemnt le liquide contient de l'eau oxygénée,
- de l'eau acidulée de potentiel Hydrogène inférieur à sept (pH<7),
- de l'eau chargée et,
- de l'eau additionnée d'un ou de polluants.

La nature du ou des polluants est choisie parmi le groupe de polluants comportant :
- l'ozone,
- l'anhydride carbonique,
- les oxydes d'azote,
- les anhydrides sulfuriques et sulfureux,
- les chlorures,
- les composés de fluor,
- les composés de phosphore,
- les insecticides,
- les pesticides et,
- les aérosols.

### BREVE DESCRIPTION DES DESSINS

D'autres objectifs, caractéristiques et avantages de l'invention apparaîtront à la lecture de la description ci-après d'un mode de réalisation, donné uniquement à titre d'exemple non-limitatif, description intégrant les dessins annexés sur lesquels :
- la figure 1A représente schématiquement une vue en coupe axiale de dispositif d'exposition à des rayonnements selon l'invention,
- la figure 1B représente schématiquement une vue de face en coupe transversale du dispositif selon l'invention,
- Les figures 2B, 2A et 2C représentent des vues d'une réalisation de dispositif selon l'invention, avec capot escamoté, la figure 2A montrant le dispositif sans capot et sans porte-lampe, vu en coupe axiale, la figure 2B montrant le capot escamoté, en vue de trois quart vers l'intérieur, la figure 2C montrant la chaîne cinématique du dispositif, vue en coupe transversale vers l'arrière suivant le tracé C-C de la figure 2A.
- les figures 3A et 3B représentent en vue latérale et en coupe transversale les détails d'une forme de réalisation avantageuse de capot pour dispositif selon l'invention,
- les figures 4A, 4B et 4C représentent en vue latérale, en vue de face et en vue cavalière une forme de réalisation avantageuse de cage porte-échantillons pour dispositif selon l'invention,
- la figure 5A représente une vue en demi-coupe axiale et en demi-profil d'une réalisation avantageuse de mécanisme d'entraînement débrayable pour cage porte-échantillons de dispositif selon l'invention,
- la figure 5B représente une vue en demi-coupe transversale et de face du mécanisme d'entraînement débrayable de la figure 5A, et

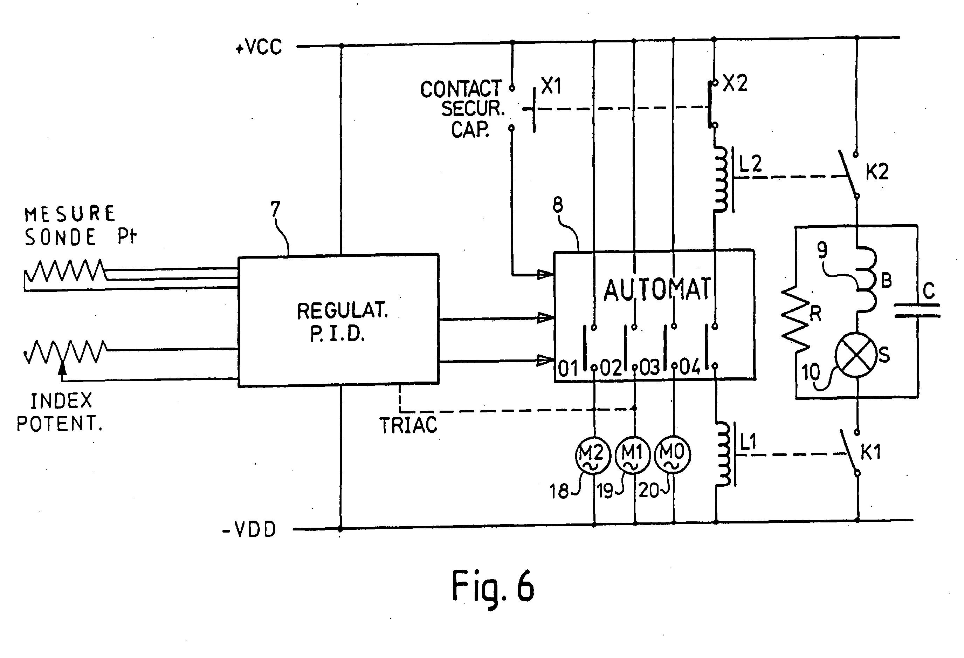

- la figure 6 représente un schéma de principe de circuit d'alimentation électrique et d'asservissement en température pour dispositif selon l'invention.

### DESCRIPTION DETAILLEE DES MODES DE REALISATION PREFERES

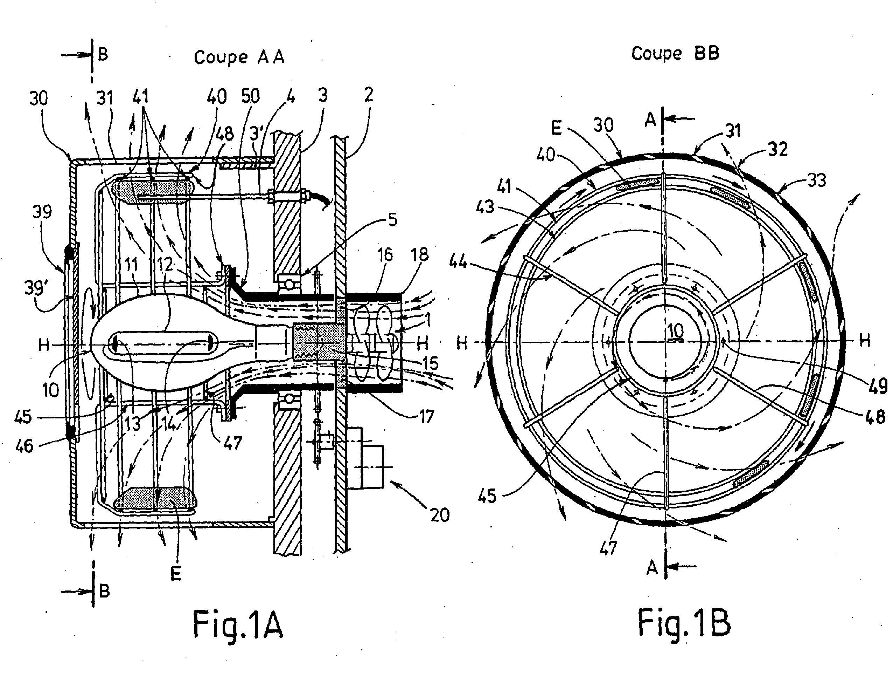

Sur les figures 1 à 5, il apparaît clairement que le dispositif selon l'invention est, de préférence, globalement circulaire et symétrique de révolution autour d'un axe H-H destiné à être disposé avantageusement dans une direction horizontale.

Dans la description du mode de réalisation et de fonctionnement ci-après, on considérera pour simplifier que le dispositif est symétrique de révolution autour de l'axe H-H, des géométries plus complexes pouvant être envisagées par l'homme du métier sans sortir du cadre de la présente invention.

La figure 1A montre ainsi que le dispositif selon l'invention comporte une enceinte 3, 30 cylindrique et circulaire. Alternativement, le dispositif peut comporter une enceinte cylindrique mais de section carrée, rectangulaire ou autre. L'enceinte comporte un capot 30 amovible de préférence en forme de cuve, qui vient se fixer hermétiquement sur une platine 3 fixe. L'enceinte 3, 30 contient une cage porte-échantillons 40 de forme circulaire qui peut être réalisée selon l'exemple des figures 1A, 2A et 4, à partir d'une armature de tiges métalliques 41 à 48 cintrées en forme d'éléments circulaires 41,43,45,47,49 et d'éléments recourbés 48 disposés en quartiers et solidarisés entre eux.

La cage 40 est solidarisée avec un moyeu 50-51, par l'intermédiaire d'une pièce d'entraînement circulaire 49 et d'un mécanisme débrayable 55,56,57,58,59.

Le moyeu 50-51 est monté en rotation, éventuellement sur roulement 5 à billes ou à rouleaux, sur la ou les platines 2-3 qui offrent un support fixe.

Le moyeu 50 est entraîné en rotation par une chaîne cinématique comportant un moteur désaxé 20 relié par une transmission 21, 22 à engrenage ou à chaîne.

Selon le mode de réalisation préféré de l'invention, le moyeu 50-51 est complètement évidé axialement, l'évidement formant une conduite d'arrivée de flux d'air entrant en direction de l'enceinte.

De même selon le mode de réalisation préféré, la partie centrale de la cage porte-échantillons 40 est évidée, aucune tige de l'armature n'apparaissant dans la partie centrale 45, 46, 47 de la cage qui correspond à la prolongation de la partie évidée du moyeu 50.

En effet, l'invention prévoit de disposer fixement une ou des lampes 10 à rayonnement électromagnétique dans la partie centrale de la cage rotative 40.

La ou les lampes 10 sont raccordées, c'est-à-dire fixées et alimentées électriquement, directement par l'intermédiaire d'une pièce axiale 15 ayant des branches radiales 16, 17 en forme d'aster reliée à la platine 2 de support fixe, ou indirectement par l'intermédiaire du corps 18 du ventilateur 1.

Si une seule lampe à ultraviolet 10 équipe le dispositif selon l'invention, il est préférable que la lampe 10, généralement tubulaire ou symétrique de révolution soit positionnée dans la partie centrale 45, 46, 47 de la cage, l'axe de la lampe étant disposé dans l'axe H-H de la cage 40.

Si deux ou plusieurs lampes équipent le dispositif selon l'invention, il est préférable que les lampes soient disposées symétriquement autour de l'axe de la cage et parallèlement audit axe H-H.

De façon particulièrement avantageuse, la disposition de la ou des lampes 10 dans la partie centrale de la cage porte-échantillons 40 sur ou autour de l'axe de rotation H-H, permet d'exposer les échantillons en rotation à un flux de lumière, parfaitement homogène (à condition que les échantillons soient disposés parallèlement et à la même distance radiale de l'axe).

Il est encore prévu, selon l'invention, que le dispositif comporte des moyens d'insufflation d'air 1 disposés dans le prolongement de l'évidement du moyeu 50.

On peut prévoir de monter un ventilateur 18 dans le prolongement arrière de l'évidement du moyeu 50 pour aspirer l'air extérieur et refouler le flux d'air à l'intérieur de l'enceinte 30 à travers la conduite d'air creusée dans le moyeu 50.

Le ventilateur 18 peut être fixé sur la platine arrière 2 fixe.

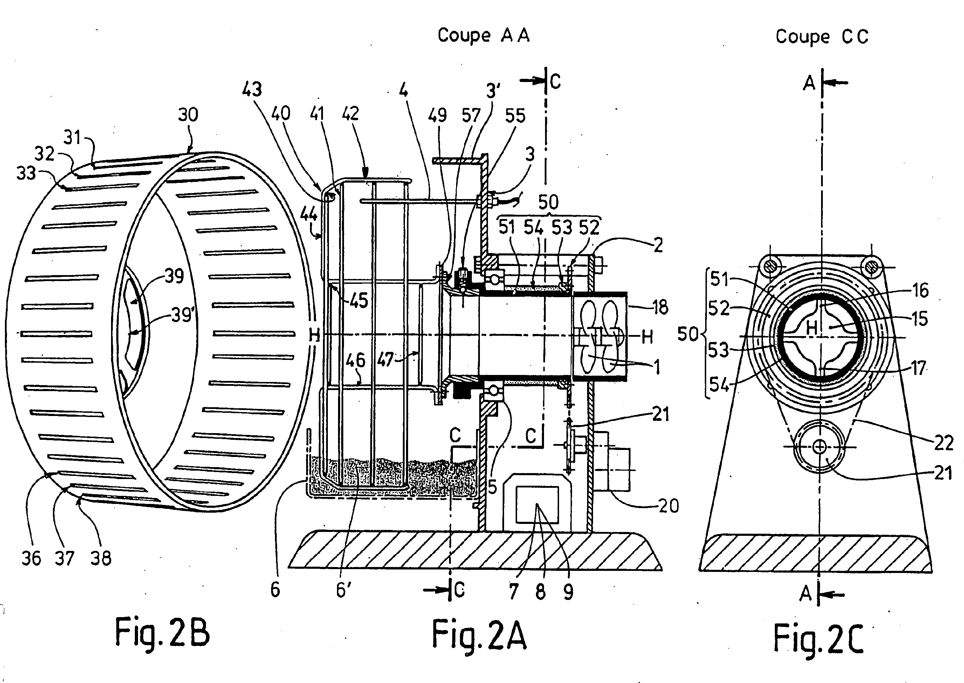

Selon l'exemple de réalisation des figures 1A et 2A, il est prévu de monter deux ventilateurs 18 et 19 en série à l'arrière de l'évidement du moyeu 50. Le second ventilateur forme avantageusement un ventilateur d'appoint ou de secours.

L'intérêt de cette disposition est d'augmenter la puissance sur plusieurs niveaux et d'augmenter la fiabilité en cas de défaillance d'un ventilateur.

Cette disposition permet d'insérer un filtre à air (non représenté) destiné à retenir les particules entraînées par le flux d'air avant d'arriver dans l'enceinte 30.

De façon avantageuse, les parties centrales de la cage 40 et du moyeu 50, ainsi que leurs prolongements sont complètement dégagés, à l'exception du porte-lampe 15, pour laisser passer librement le flux d'air.

Ainsi, selon l'invention l'arrivée du flux d'air se fait dans la direction axiale H-H au centre de la cage 40, le dispositif comportant avantageusement une cage 40 et un moyeu 50 dont la partie centrale est complètement évidée.

D'autre part, l'invention prévoit de disposer des ouvertures d'évacuation d'air 31,32,33,...,36,37,38 à la périphérie de la cage 40.

Selon le mode de réalisation préféré de l'invention, l'enceinte cylindrique 30 comporte ainsi des ouïes 31 à 38, c'est-à-dire des ouvertures de sortie d'air, percées sur le pourtour cylindrique ou quasi-cylindrique (légèrement conique), du capot 30 ou plus généralement de l'enceinte.

Lors des tests, il est prévu de disposer les échantillons E à la périphérie de la cage, en les fixant sur les éléments circulaires 41 de l'armature de la cage, notamment à l'aide de pinces.

On considère communément que les échantillons sont découpés en plaques ou en cartes, de forme planaire en général, si bien que les échantillons viennent occuper des surfaces sécantes ou tangentielles à la périphérie de la cage circulaire 40, comme illustré figure 1B.

En fonctionnement, les échantillons E sont donc en position tangentielle ou sécante à la périphérie de la cage 40, la cage est mise en rotation et les moyens 1 d'insufflation d'air amorcent un flux d'arrivée d'air dans l'axe H-H de la cage 40, de la lampe 10 et du moyeu 50.

Suivant le perfectionnement de l'invention, l'air arrive donc directement sur la lampe en suivant une direction axiale, donc sensiblement horizontalement avant d'atteindre la partie centrale de la cage 45, 46, 47.

Le flux d'air refroidit alors avantageusement la lampe à arc dont la température atteint couramment une température de l'ordre d'une à quelques centaines de degrés Celsius.

Il est possible d'adopter avantageusement des lampes à vapeur de Mercure moyenne pression ou encore des lampes dites H.M.I., (abréviation de l'anglais "*Metal Halide*"), c'est à dire des lampes à arc dont la vapeur est formée d'un alliage ou d'un composé d'halogénure métallique, notamment d'iodure et de mercure.

Ces lampes ont l'avantage majeur de fonctionner à faible puissance relativement aux lampes à Arc ou Xénon. Les ampoules de lampes à vapeur de Mercure ou de composés Mercuriel ont des dimensions réduites et existent avec des culots compatibles sur les douilles E40, suivant le standard européen très répandu qui accepte aussi les lampes au Sodium et les lampes à incandescence classiques.

Les avantages essentiels des sources lumineuses constituées de lampe à arc au mercure ou d'halogénure métallique est d'avoir un rendement très élevé, un spectre bien reparti avec un équilibre entre les U.V.A. et les U.V.B., très peu d'émission d'Infrarouges et par suite peu de dégagement de chaleur.

Ces propriétés particulièrement intéressantes en font des lampes idéales pour des appareillages de test de vieillissement accéléré. Un avantage essentiel est que leur faible dégagement de chaleur autorise de les employer nues, contrairement aux sources de l'art antérieur.

Consécutivement le flux d'air arrivant dans l'enceinte se réchauffe au contact de la lampe 10 en absorbant la puissance calorique dégagée, ce qui permet d'atteindre couramment des températures moyennes de l'ordre de plusieurs dizaines à une ou deux centaines de degrés Celsius.

Typiquement, dans les conditions ambiantes d'un laboratoire à 28°C, le flux d'air extérieur entrant dans l'enceinte peut ainsi s'échauffer au contact de la lampe pour atteindre une température de l'ordre de quelques dizaines à une centaine de degrés Celsius.

Ce résultat constitue un avantage majeur de l'invention, car les normes de test de photovieillissement préconisent d'effectuer les essais polymères en les portant à des températures d'environ 63°C ou de 83°C ou encore entre 80°C et 100°C pour se replacer dans des conditions proches de celles régnant dans l'habitacle d'un véhicule automobile exposé au soleil.

L'avantage primordial de l'invention est de fournir ainsi un système de circulation d'air simple qui combine l'évacuation de la chaleur dégagée par la lampe avec la régulation de la température des échantillons en les portant à une température optimale pour accélérer les tests d'échantillons polymères puis en maintenant étroitement cette valeur de températures, une fois le régime de fonctionnement établi.

Il faut noter que le dispositif met en oeuvre des lampes de faible puissance de l'ordre de plusieurs dizaines à plusieurs centaines de Watts, typiquement 250W ou 400W, si bien que la quantité d'énergie calorique à évacuer est relativement faible et que la température reste stable.

Comparativement les dispositifs de l'état de la technique utilisant des lampes à arc au Xénon ou des torches à arc au Carbone ont un faible rendement, ce qui nécessite une puissance électrique élevée et d'imposants équipements de refroidissement qui provoquent immanquablement une instabilité et de fortes variations de température d'échantillons au cours de test.
L'adoption de lampes à arc faible puissance, comme les lampes à vapeur de Mercure moyenne pression, permet avantageusement d'effectuer un refroidissement par air de la lampe et de réutiliser directement ce flux d'air réchauffé pour amener et maintenir les échantillons à la température de test.

L'invention prévoit ainsi que le flux d'air diverge autour de la lampe en direction perpendiculaire à l'axe H-H pour rejoindre directement les échantillons et les tempérer.

Il est prévu que le flux d'air s'évacue alors par les sorties d'air à la périphérie de l'enceinte 30. En s'échappant, le flux d'air vient frapper les échantillons E orthogonalement ou avec un angle d'incidence par rapport à leur plan, comme illustré figure 1B.

De façon avantageuse, l'incidence du flux d'air sur les échantillons E améliore les échanges thermiques.

Contrairement aux dispositifs de l'état de la technique, les échantillons E ne sont donc pas longés par un flux d'air laminaire, mais sont soumis à un flux d'air incident et tourbillonnaire.

Dans le cadre de certaines réalisations, le flux de sortie d'air peut être strictement radial au niveau des échantillons E disposés à la périphérie de la cage 40.

Ce cas se présente en particulier lorsque chaque ouïe de sortie d'air 31, 32, 33,... est percée radialement dans la partie cylindrique de l'enceinte 30, à condition que la vitesse de rotation de la cage 40 ne modifie pas la circulation d'air.

Dans le cadre d'autres réalisations, le flux d'entrée d'air à la périphérie de la cage porte-échantillons 40, comporte à la fois une composante radiale et une composante tangentielle, si bien que les échantillons sont soumis à un flux d'air ayant un certain angle d'incidence.

Dans le mode de réalisation préféré, il est ainsi prévu de percer des ouïes non-radiales 31 à 38 à la périphérie de l'enceinte cylindrique 30.

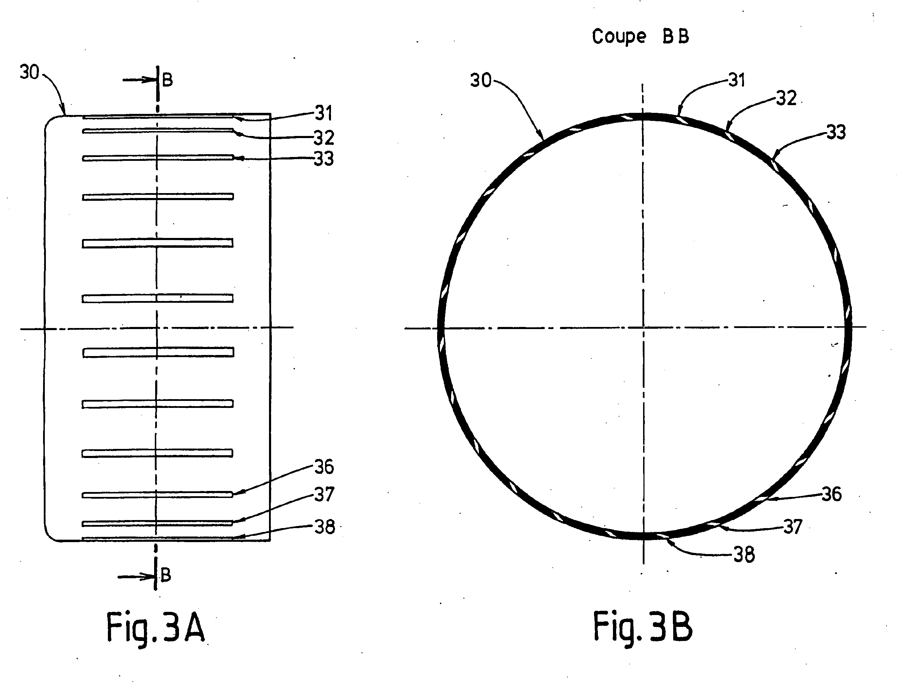

Les figures 3A et 3B montrent ainsi que le pourtour quasi-cylindrique du capot 30 de l'enceinte est entaillé par de fines ouvertures 31,32,33,...,36,37,38 formant des fentes tracées axialement, ces fentes ayant la particularité de ne pas s'enfoncer suivant un plan radial, mais suivant une direction oblique par rapport à la direction radicale ; à la limite les ouvertures 31 à 38 peuvent être quasiment tangentielles à la partie circulaire du capot 30.

De façon avantageuse, les ouïes obliques 31 à 38 permettent au flux d'entrée d'air d'avoir des composantes tangentielles et radiales au niveau des échantillons E disposés à la périphérie de la cage 40.

La circulation d'air est ainsi avantageusement analogue à un mouvement d'anticyclone autour de l'axe de la cage, ce qui assure des échanges thermiques optimums au niveau des échantillons.

Un autre avantage essentiel de la disposition oblique des ouïes d'entrée d'air 31 à 38 à la périphérie de l'enceinte cylindrique 30 est d'obturer la sortie directe des rayonnements de la lampe 10 tout en permettant le passage de l'air de façon analogue au voletage d'un store ou d'un volet.

Dans le mode de réalisation préféré de l'invention, chaque fente d'ouïe 31 a une ouverture réduite, mais un angle d'inclinaison élevé par rapport à la direction radiale ainsi qu'une profondeur élevée, de sorte que le rayonnement direct de chaque lampe 10 positionnée dans la partie centrale de la cage ne traverse pas l'ouïe 31 et ne s'échappe pas de l'enceinte.

D'autre part, il est prévu optionnellement, comme illustré sur la figure 2A, que le capot 30 comporte un hublot central 39 en verre anti-U.V..

Ce hublot 39 dont le verre coupe les rayonnements ultraviolets permet avantageusement d'observer les échantillons en cours de test, en évitant les rayonnements nocifs de la lampe.

De façon avantageuse, la partie centrale du hublot 39 peut être masquée par une pièce centrale 39' fixée au capot 30 par des branches radiales en forme d'aster comme le montre la figure 2B.

Le capot 30 est réalisé de préférence en tôle, par emboutissage, ce qui donne un capot de forme légèrement conique, quasi-cylindrique.

Une telle réalisation légère a l'avantage de limiter le prix de l'équipement et des investissements de tests de vieillissement.

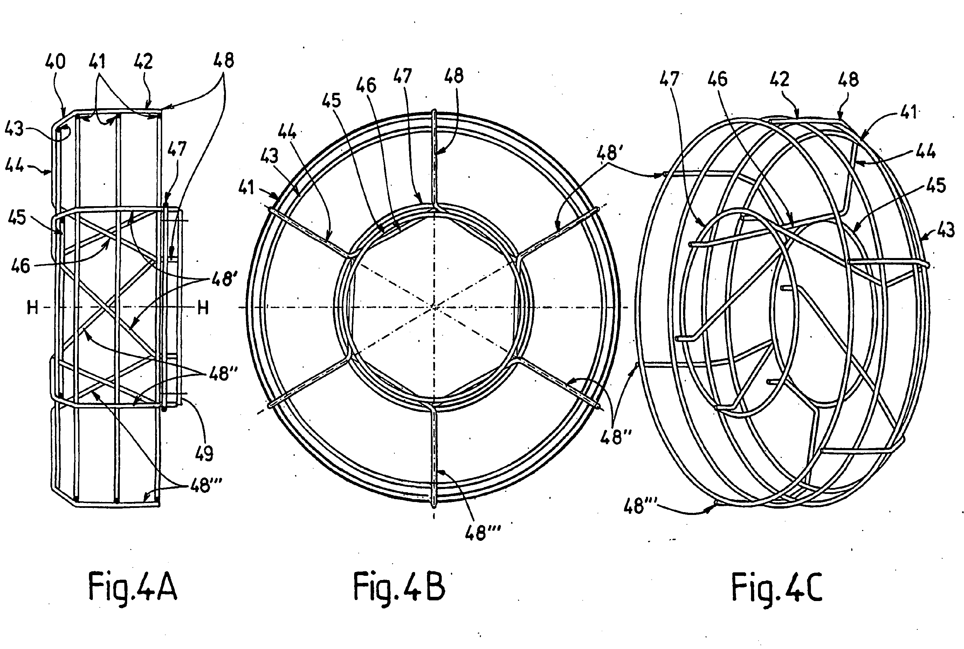

Les figures 4A et 4B montrent que la cage 40 du dispositif est réalisée de préférence à partir de fils ou de tiges métalliques semi-rigides 41 à 48.

Le principal avantage d'une telle réalisation est de limiter le poids de la cage 40, donc le dimensionnement du moteur d'entraînement 20.

Une telle réalisation facilite également la circulation d'air et la manipulation des échantillons.

Cette réalisation légère a aussi l'avantage de limiter le coût de l'équipement.

Dans la première forme de réalisation, illustrée sur les figures 1A, 1B et 2A, la cage 40 a une armature particulière en forme de parapluie.

L'armature est formée de tiges recourbées 48 disposées en quartier suivant des plans radiaux de façon à rayonner autour de la partie centrale évidée de la cage 40.

Comme le montre la figure 4A, chaque tige recourbée 48 comporte un brin central 46 s'étendant parallèlement à l'axe H-H à la limite de la partie centrale évidée de la cage 40. Le brin central 46 se prolonge par un brin 44 s'étendant dans la direction radiale à l'opposé du centre. Le brin radial 44 est lui-méme relié ou prolongé par un brin périphérique 42 parallèle à l'axe, qui délimite la périphérie de la cage 40.

La particularité de l'armature de cage 40 des figures 1A à 2A est que le brin central 46 et le brin périphérique 42 de chaque tige recourbée 48 sont situées dans le même demi-espace délimité par le plan défini par les brins radiaux 44. Chaque tige recourbée 48 a donc une forme de crochet.

Par la suite, des tiges circulaires 41 et 43 sont fixées, notamment par soudure, sur les brins périphériques 42 des tiges recourbées 48 pour former l'armature de la cage circulaire 40. De même, des tiges circulaires de petit diamètre 45 et 47 peuvent être solidarisées avec les brins centraux 46 des tiges recourbées 48 pour achever la formation de l'armature et augmenter avantageusement la rigidité de la cage circulaire 40.

De préférence, suivant la forme de réalisation illustrée sur les figures 1A et 2A, la cage comporte des éléments circulaires 41 et 43 de diamètres différents pour constituer une armature multi-positions pour les échantillons. Ainsi, des échantillons plans peuvent être positionnées parfaitement parallèle à l'axe H-H en les fixant aux éléments 41 de même diamètre ou bien en position oblique par rapport à l'axe en les fixant d'un côté à un élément 41 de grand diamètre et d'un autre côté à un élément 43 de petit diamètre.

De façon avantageuse, cette armature multi-positions permet de disposer des échantillons de longueurs différentes sous des angles différents d'incidence de rayonnement et à des distances différentes de la source lumineuse, ce qui permet d'exposer des échantillons de longueurs différentes à la même puissance surfacique lumineuse.

Enfin, les brins centraux 46 de l'armature de la cage sont fixées par collage, par soudure, attaches ou tout autre moyen, sur une pièce en forme de rondelle 49 destinée à être solidaire du moyeu 50.

Une telle armature de cage en parapluie permet avantageusement de disposer des instruments 4 fixement au sein de la cage rotative.

Les instruments, de type capteurs ou actionneurs, sont fixés à la platine 3 de support fixe du dispositif et font saillie de la platine (parallèlement ou obliquement par rapport à l'axe H-H) pour s'engager au sein de la cage rotative 40 sans affecter la rotation.

Ainsi, il est prévu avantageusement de fixer une sonde de température 4 sur la platine 3, au-dessus de la lampe axiale 10, l'extrémité active de la sonde s'avançant entre la lampe 10 et les échantillons E sans empêcher alors la rotation de la cage en forme de parapluie.

Autre exemple (non-illustré), il est prévu de disposer une rampe d'aspersion d'eau en dessous et parallèlement à la lampe axiale 10 en la fixant à la platine 3 pour la relier à une arrivée d'eau.

De façon avantageuse, la rampe est ainsi immobilisée au sein de la cage rotative parallèlement au plan des échantillons E, qui sont alors aspergés régulièrement sur toute leur surface à chaque cycle de rotation.

En outre, selon une forme de réalisation améliorée illustrée sur les figures 4A, 4B et 4C, il est prévu que les brins centraux 46 des tiges recourbées 48 ne sont pas dirigés parallèlement à l'axe H-H, mais s' enroulent en hélices autour de l'axe H-H de la cage 40.

L'avantage d'une telle amélioration est d'éviter qu'une partie d'un échantillon soit plongée dans l'ombre projetée par un brin central 46 d'une tige recourbée 48. Le rayonnement électromagnétique de la lampe centrale atteint ainsi uniformément chaque échantillon, quelle que soit sa position à la périphérie de la cage.

Maintenant du point de vue mécanique, la cage 40 est entraînée en rotation par une chaîne cinématique 50 qui comporte des dispositions particulières, comme illustré sur les figures 2A et 2C.

Il est prévu avantageusement que le moteur d'entraînement 20 est désaxé au lieu d'être situé dans l'axe H-H de la cage 40.

Ceci permet de dégager complètement la portion centrale évidée du dispositif et son prolongement, pour ne pas gêner la circulation d'air.

Le moteur 20 est fixé à une platine 2 de support fixe. Le pignon de sortie 21 du moteur entraîne une couronne ou un plateau denté 52 fixé au moyeu 50-51.

La transmission peut se faire soit directement par engrenage, le pignon 21 et le plateau 52 dentés étant en contact, soit indirectement par l'intermédiaire d'une chaîne, ou d'une courroie 22 tendue entre un pignon 21 et une couronne 52 à gorge, comme illustré figure 2C, ou encore par tout autre moyen de transmission.

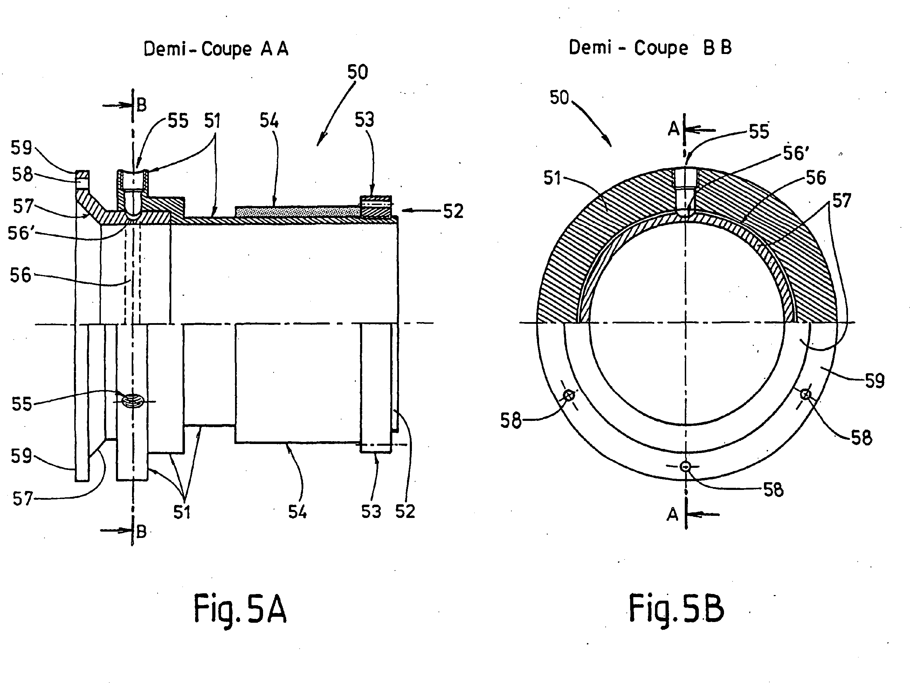

Dans l'exemple de réalisation des figures 2A et 5A, la couronne ou le plateau d'entraînement 52 est attaché par des vis sur un écrou 53 bloqué sur le pas de vis du moyeu 51.

L'écrou 53 permet en outre de bloquer une entretoise 54 cylindrique contre la bague du roulement à billes 5 ou le pourtour circulaire de la platine 3.

Comme l'autre extrémité 55 du moyeu est évasée, le moyeu 51 est alors immobilisé à rotation par rapport à la platine 3 de support fixe.

Enfin, de façon particulièrement avantageuse, il est prévu que la cage 40 est reliée au moyeu 51 par l'intermédiaire d'un mécanisme débrayable 50.

Le moyeu 50 est ainsi constitué de deux corps concentriques 57 et 51 à extrémités évasées 59 et 55, emboîtés l'un dans l'autre.

Comme l'illustre la figure 5A, le corps 57 du moyeu 50 a une forme évasée en entonnoir s'achevant par une extrémité 59 circulaire plane, creusée de pas de vis 58 pour fixer la cage 40 par l'intermédiaire de la rondelle 49.

L'autre extrémité, cylindrique, du corps 57 du moyeu 50 est creusée d'une gorge annulaire 56 sur tout le pourtour.

En outre, la gorge annulaire 56 est creusée de plusieurs cavités hémisphériques 56' en plusieurs points de son pourtour pour approfondir la dépression de la gorge 56. Selon l'exemple de la figure 5, il est prévu ainsi de disposer trois cavités 56' de façon équidistante, à cent-vingts degrés l'une de l'autre, sur le pourtour de la gorge annulaire 56.

Corrélativement, l'extrémité évasée du corps principal 51 du moyeu 50 est percée radialement de logements tubulaires 55, correspondant aux cavités 56' d'approfondissement de la gorge 56 du corps d'extrémité 57.

Chaque logement 55 est destiné à recevoir une bille et un ressort maintenus par une vis de réglage en pression.

Lorsque le corps d'extrémité 57 est emboîté dans le corps principal 51 du moyeu 50, chaque bille vient donc s'engager dans la gorge annulaire 56, puis se bloque au fond de la cavité hémisphérique 56' correspondante.

Comme les billes sont maintenues au fond des cavités 56' creusées dans la gorge 56, ce mécanisme permet de bloquer en translation et en rotation le corps 57 par rapport au corps 51 du moyeu, de façon analogue au clavetage d'un barillet dans un cylindre de serrure.

Cependant si un couple excessif est exercé sur le corps 57 par rapport au corps 51 du moyeu 50, les billes sont repoussées hors de leurs cavités 56' respectives et le corps 57 se met à tourner librement autour du corps 51, les billes restant engagées dans la gorge annulaire 56.

Ce mécanisme permet très avantageusement un débrayage en rotation de la cage 40 par rapport au moyeu 50, lorsqu'il apparaît un couple axial excessif entre la cage et le moyeu.

De façon avantageuse, ce mécanisme débrayable permet à un opérateur de tourner la cage 40, alors que le moteur 20 est à l'arrêt.

De même, ce mécanisme évite une atteinte du moteur 20 lorsque la cage 40 se bloque accidentellement en rotation.

En outre, ce mécanisme permet très avantageusement un découplage en translation entre la cage 40 et le moyeu 50 lorsqu'on effectue une traction axiale excessive sur la cage.

En effet, en exerçant une traction sur le corps d'extrémité 57 dans la direction axiale, vers l'avant, les billes finissent par s'échapper du lit de la gorge annulaire 56 et le corps d'extrémité 57 se désolidarise du corps principal 51 du moyeu 50.

Ce mécanisme permet donc d'escamoter ou de débrayer en translation la cage du dispositif.

Enfin, il est prévu avantageusement que chaque logement 55 comporte une extrémité resserrée à un diamètre inférieur au diamètre des billes, pour éviter aux billes de s'échapper des logements 55 lorsque le corps 57 est désolidarisé.

Par ailleurs, il est prévu selon un perfectionnement essentiel que le dispositif selon l'invention comporte un réservoir d'eau baignant la portion inférieure de la cage rotative.

Un bassin 6 en forme de demi-lune peut ainsi être fixé au bas de la platine 3 du dispositif, la cage 40 étant plongée partiellement dans l'eau 6' remplissant lé bassin 6.

Une telle disposition permet de tester la tenue des échantillons au vieillissement sous l'action combinée de l'humidité, de l'immersion dans l'eau et de rayonnements photoniques comme les ultraviolets, ce qui rassemble avantageusement la plupart des agents naturels de vieillissement, comme ceci apparaît dans les développements du perfectionnement de l'invention qui mènent au meilleur mode de réalisation de l'invention qui va être décrit maintenant.

### DESCRIPTION DETAILLEE DU MEILLEUR MODE DE REALISATION

Ainsi, suivant le meilleur mode de réalisation de l'invention, une cuve (c'est-à-dire un bassin ou un réservoir) destinée à contenir un liquide, notamment de l'eau et diverses compositions acqueuses, est disposée à l'intérieur de l'enceinte 30 de sorte que la cage porte-échantillons 40 baigne partiellement dans le liquide contenu dans la cuve.

La cuve est solidement fixée à la platine 3 de cloison dans la partie basse de l'enceinte.

La cuve a de préférence la forme d'un bassin en demi-lune pour épouser l'enceinte 30 et la cage 40 cylindriques. D'autres conformations peuvent être adoptées selon la forme de l'enceinte.

La cuve a une dimension transversale et une profondeur verticale suffisante pour envelopper une portion assez importante de la cage 40.

Typiquement, la hauteur utile H de la cuve est de l'ordre du tiers de la dimension radiale R de la cage, mais la hauteur utile H peut atteindre des valeurs supérieures jusqu'à la valeur du rayon R de la cage.

Une telle hauteur permet d'immerger une portion inférieure de la cage 40 correspondant à un secteur angulaire de 120°, voire des valeurs supérieures jusqu'à approcher 180° d'angle.

La cuve est dotée d'un système de remplissage et d'évacuation qui peuvent être montés commodément à travers la cloison verticale de la platine 3 de l'enceinte. Le système peut comporter par exemple une buse d'arrivée d'eau ou d'autre liquide, tandis que le fond de la cuve est relié par une bande à une conduite d'évacuation.

On peut prévoir que la conduite d'arrivée de liquide soit reliée par une vanne multivoies à plusieurs conduites pour mélanger des adjuvants dans le liquide comme la suite en fait état.

Cumulativement ou alternativement, une rampe d'aspersion peut être montée au-dessus de la cuve pour asperger les échantillons et remplir la cuve.

Il est enfin prévu une jauge de niveau de liquide dans la cuve, divers principes de jauges de niveau à flotteur, à mesure de résistivité, etc. étant à la portée de l'homme de métier.

En fonctionnement en effet, il est prévu de maintenir un niveau constant de liquide dans la cuve pour tremper les échantillons E1-E9 durant la périodes de temps déterminée.

Ainsi, le remplissage de la cuve sur une hauteur utile P conséquente permet d'immerger un secteur inférieur de la cuve d'environ 120° correspondant au tiers du périmètre de la cage 40.

Lors de la rotation de la cage, les échantillons E1-E9 alternent alors des phases d'immersion dans le liquide de la cuve avec des phases d'émersion dans le volume supérieur de l'enceinte 30, où ils sont exposés au flux d'air. Les échantillons E1-E9 sont ainsi exposés à l'immersion pendant un tiers de la durée des essais.

On obtient ainsi une suite d'alternances de périodes d'exposition sèche et de périodes d'exposition des échantillons à l'immersion, qui correspond très naturellement à des cycles météorologiques réels.

En faisant varier le niveau de liquide par un système de commande adéquat, on fait varier avantageusement la durée et proportion des phases d'immersion des échantillons.

Pratiquement, la durée de la phase immergée peut varier dans des proportions allant d'un dixième ou même de quelques centièmes à un tiers de la durée du cycle de rotation de la cage.

La durée des phases immergées et émergées des échantillons dépend de la vitesse de rotation de la cage.

En pratique, la vitesse de rotation de la cage est relativement faible, voire très faible, puisqu'elle est de l'ordre d'un dixième de tour par heure à dix tours par heure, en tous cas inférieure à un tour par minute.

De façon avantageuse, ces vitesses permettent aux phénomènes de dégradation recherchés de s'établir étant donné que les cycles circadiens et les périodes de pluies sont du même ordre de grandeur.

La norme ISO 4892.3 suggère ainsi des cycles d'exposition sèche de 8 heures alternant avec des cycles d'exposition à la condensation de 4 heures.

L'intérêt du dispositif est de pouvoir faire varier les facteurs de dégradations que sont l'exposition au flux d'air de l'enceinte et l'immersion dans le liquide de la cuve sur de grandes latitudes, ce qui permet avantageusement de comparer et de corréler les résultats de vieillissement artificiel accéléré avec des résultats de vieillissement naturel et de valider des protocoles d'essais pertinents. Ce n'est pas l'objet de la présente de définir ces protocoles qui doivent faire l'objet d'études et de normes pour l'homme de l'art.

L'autre intérêt essentiel du dispositif perfectionné selon l'invention, est de permettre non seulement de reproduire, mais surtout d'additionner et de combiner divers facteurs de dégradation connus qui habituellement font l'objet d'expérimentation séparées.

Ainsi, l'alternance d'exposition sèche photonique et d'immersion des échantillons peut être suivie d'une autre alternance d'exposition sèche dans l'obscurité et d'immersion.

Mais surtout, on peut constater que lors des phases d'immersion, l'exposition photonique continue.

les deux facteurs de dégradation connus, l'humidité et l'éclairement sont donc combinés simultanément.

En effet, les rayonnements U.V. émis par la lampe à arc traversent l'eau quasiment sans absorption.

Par suite, lorsque les échantillons sont dans la cuve, sous une profondeur d'eau P, ils sontinuent à être exposés aux rayonnements photoniques de la source centrale, en particulier aux rayonnements ultra-violets.

Or, il est connu que certaines mécanismes de dégradation recquiérent la présence simultanée de plusieurs facteurs pour se perpétuer.

Selon les modèles connus en effet, les mécanismes de dégradation des polymères comportent une étape d'initiation dans laquelle intervient l'énergie lumineuse des photons UV.

Mais, pour que le mécanisme s'enchaîne et se perpétue, il faut que la réaction d'initiation soit immédiatement suivie de réactions de propagation qui font intervenir l'oxygène. L'oxygène est en particulier fourni par l'eau sous forme d'oxygène dissous.
Ces réactions de propagation s'achèvent par des réactions de terminaison où intervient la possibilité de réticulation entre des chaînes de polymères.

Ce sont les réactions de réticulation qui affectent le plus la couleur, l'aspect et les propriétés des polymères. Ces réactions sont donc recherchées lors des campagnes de tests.

En absence d' eau et d'oxygéne, l'étape d'initiation s'amorce sans conséquence et les réactions de propagation ne s'enchainent pas. Le mécanisme de dégradation reste alors en sommeil.

Avec le dispositif perfectionné selon l'invention, on peut ainsi de façon essentielle combiner une exposition aux rayonnements photoniques avec une immersion dans l'eau et faire surgir ces mécanismes de dégradation.

Il est donc particulièrement avantageux de pouvoir combiner une exposition aux rayonnements photoniques avec une exposition à l'immersion.

L'eau apparait ainsi comme un activateur d'oxygène. C'est pourquoi les tests normalisés prévoient des phases d'exposition à l'humidité généralement effectué par condensation ou aspergé par des gicleurs selon les dispositifs de l'état de la technique.

Les normes ISO-4892 préscrivent d'utiliser de l'eau pure.

Cette disposition correspond aux possibilités des dispositifs connus puisque le fonctionnement des gicleurs recquiert d'utiliser de l'eau ne contenant pas d'impuretées et que la condensation se forme également avec de l'eau pure.

Contrairement à cet usage, le dispositif selon l'invention offre la possibilité de tester des milieux liquides qui accélérent avantageusement les phénomènes de dégradation.

Ainsi, de façon inusitée, il est prévu, selon l'invention, de procéder à des protocoles d'essai dans lesquels le liquide peut être de l'eau contenant de l'oxygène dissous, ou un liquide contenant de l'eau oxygénée.

L'invention permet ainsi de tester l'exposition des échantillons non seulement à l'eau pure mais à d'autres liquides.

Il est préférable de ne pas utiliser de l'eau oxygénée non-coupée, ni de grande concentration d'eau oxygénée. L'eau oxygénée est un activateur excessivement puissant qui risquerait de faire apparaitre des phénomènes de dégradation inobservés dans la réalité.

Ainsi, la phase d'immersion se fait dans un bain d'eau contenant une proportion d'eau oxygénée.

Accessoirement, comme l'eau oxygénée peut se décomposer sous l'effet des UV, on peut se servir du cache de protection contre l'insolation pour protéger le bain de liquide contenant l'eau oxygénée contre le rayonnement UV.

Dans la pratique, la décomposition de l'eau oxygénée ne s'observe pas.

Il y a donc avantage à cumuler en une seule phase, une exposition combinée à l'immersion dans l'eau partiellement oxygénée et aux rayonnements photoniques, - pour observer les phénomènes de dégradations précités.

Enfin, de façon avantageuse, le procédé de test perfectionné selon l'invention prévoit avantageusement des protocoles d'essais dans lesquels le liquide est de l'eau acidulée.

Ce protocole permet avantageusement de reproduire l'exposition climatique aux pluies acides.

Le procédé permet encore d'effectuer des protocoles d'essais dans lesquels le liquide est de l'eau chargée de particules.

Ces dispositions permettent de tester l'interaction de matériaux solides intervenant dans les phénomènes de dégradation.

L'utilisation d'une cuve de liquide permet avantageusement de tester l'interaction avec des particules solides, facteurs de dégradations qui sont prohibés dans les systèmes d'aspersion et les sytèmes à condensation connus dans l'état de la technique, ainsi que par les préconisations de la norme ISO-4892.

Enfin, le procédé de test perfectionné selon l'invention outre le champ à des protocoles d'essais avec des phases d'immersion dans lesquelles le liquide baignant la cage porte-échantillons est de l'eau additionnée de polluants.

Ainsi, à titre indicatifs, l'eau peut être additionnée de polluants choisis dans la liste suivante :
- l'ozone,
- l'anhydride carbonique,
- les oxydes de carbone,
- les anhydrides sulfureux et sulfuriques,
- les chlorures,
- les composés de fluor et de phosphore,
- les insecticides,
- les pesticides et,
- les aérosols.

Plus généralement, il est, particulièrement indiqué selon l'invention de procéder à des tests complets, dans lesquels les échantillons sont exposés aux divers protocoles d'essai exposés dans la présente.

Les protocoles peuvent être mis en oeuvre de façon successive pour englober tous les phénomènes de dégradation.

Bien mieux, l'invention prévoit de combiner simultanément plusieurs de ces protocoles d'essais entre eux pour observer les mécanismes de dégradation réels.

La présente demande ne se limite donc pas aux seuls protocoles décrits, mais couvre explicitement les diverses combinaisons de ces protocoles entre eux.

Pour finir, les procédés de test mettant en oeuvre le dispositif d'exposition à des rayonnements photoniques et à des agents climatiques, perfectionné selon l'invention permet ainsi de mener de façon exhaustive des essais de tous les facteurs de dégradations identifiés et recensés par la norme ISO-4892, à savoir :
- les filtres de lampe,
- le niveau d'irradiation,
- la température,
- l'exposition à l'eau,
- la température,
- l'exposition à l'eau,
- les cycles et températures d'exposition à l'eau,
- le timing des cycles d'éclairement et d'obscurité, ainsi que,
- le timing des cycles secs et mouillés.

D'autres protocoles, procédés, modes de réalisation, variantes et améliorations pourront être mises en oeuvre par l'homme de métier sans sortir du cadre de la présente invention, l'objet de la protection étant défini dans les revendications ci-après.

## Revendications

1. Dispositif d'exposition à des rayonnements photoniques pour des essais de vieillissement artificiel accéléré d'échantillons, comprenant :
- une enceinte (30),
- une cage (40) porte-échantillons rotative autour d'un axe (H-H) destiné à être sensiblement horizontal en fonctionnement,
- des moyens (15) pour positionner fixement dans la partie centrale de la cage (45, 46) et pour alimenter électriquement, au moins une lampe à arc (10) de faible puissance de l'ordre de plusieurs dizaines à un milliers de Watts, notamment une lampe à vapeur de mercure ou à vapeur de composé mercuriel, et
- un système de circulation d'air comportant un orifice (50) de passage d'air disposé dans le prolongement axial (H-H) de la partie centrale de la cage (45, 46) et plusieurs ouvertures fixes (31-38) de passage d'air disposées dans le prolongement radial de la périphérie de la cage (40), la circulation d'air étant destinée à maintenir les échantillons dans l'enceinte à une température homogène s'élevant à plusieurs dizaines de degrés Celsius,
- **caractérisé en ce que** des moyens de ventilation (1, 18, 19) insufflent l'air dans l'enceinte (30) à travers l'orifice (50) qui forme un orifice d'arrivée d'air dirigé sensiblement parallèlement à l'axe horizontal (H-H), la lampe (10) étant disposée dans le prolongement axial (H-H) immédiat de l'orifice d'arrivée d'air (50), de façon à générer un flux d'air arrivant directement sur la lampe (10) et sensiblement parallèle à l'axe dans la partie centrale de la cage (45, 46), le flux d'air divergeant autour de la lampe et s'évacuant sensiblement perpendiculaire à l'axe par les ouvertures fixes (31-38) qui forment des ouvertures de sortie d'air, à la périphérie de la cage (40), si bien que le flux d'air s'échauffe au contact de la lampe, avant d'envelopper les échantillons en élevant puis en régularisant leur température.

2. Dispositif selon la revendication 1, dans lequel le flux d'air divergeant autour de la lampe comporte essentiellement une composante sensiblement parallèle à l'axe (H-H), dans la partie centrale de la cage (45-46), auprès de la lampe (10), alors que ledit flux d'air comporte essentiellement des composantes sensiblement perpendiculaires à l'axe, à la périphérie de la cage (41) au niveau des échantillons.

3. Dispositif selon l'une des revendications 1 à 2, dans lequel l'ensemble des moyens (15) de positionnement et d'alimentation de la lampe (10), des moyens de ventilation et de circulation d'air (1, 18, 19) et des moyens d'entraînement en rotation de la cage (50) sont disposés en ligne suivant l'axe horizontal (H-H) du dispositif.

4. Dispositif selon l'une des revendications 1 à 3, dans lequel la cage (40) est montée en rotation sur un moyeu (50) évidé axialement (H-H), le flux d'air étant insufflé dans l'évidement axial du moyeu.

5. Dispositif selon la revendication 4, dans lequel le système de circulation d'air comporte des moyens de ventilation (1, 18, 19) disposés dans la lumière d'évidement du moyeu, de façon à y insuffler l'air en direction de l'enceinte (30).

6. Dispositif selon l'une des revendications 1 à 5, dans lequel le système de circulation d'air comporte une série de plusieurs ouvertures fixes (31-38) de sorties d'air disposées régulièrement à la périphérie (41) de la cage (40) avec une symétrie de révolution autour de l'axe (H-H), chaque ouverture fixe (31, 32, 33,...,38) étant orientée dans une direction comprise entre la direction radiale et la direction tangentielle à la périphérie de la cage (41).

7. Dispositif selon l'une des revendications 1 à 6, dans lequel l'enceinte cylindrique (30) comporte des ouïes (31-38) de sortie d'air disposées sensiblement à la périphérie de la cage (41), chaque ouïe (31) de sortie d'air étant dirigée non-radicalement et ayant une profondeur, une ouverture et un angle d'inclinaison par rapport à la direction radiale, tels que le rayonnement de chaque lampe (10) positionnée dans la partie centrale de la cage (40) ne traverse de préférence pas l'ouïe (31) et ne s'échappe de préférence pas directement hors de l'enceinte (30).

8. Dispositif selon l'une des revendications 4 à 7, dans lequel la cage (40) est reliée au moyeu (50) par un mécanisme d'entraînement débrayable (51,55,56,57) permettant une rotation libre de la cage.

9. Dispositif selon la revendication 8, dans lequel le mécanisme (50, 51) comporte des moyens de débrayage en rotation (55, 56, 57) sous l'effet d'un couple axial limite entre la cage (40) et le moyeu (50).

10. Dispositif selon l'une des revendications 1 à 9, comportant une cuve (6) disposée en partie inférieure du dispositif et destinée à contenir un volume de liquide de façon à baigner une portion inférieure de la cage porte-échantillons rotative, de sorte que les échantillons alternent cycliquement des phases d'immersion dans le liquide avec des phases d'émersion dans le flux d'air.

11. Dispositif selon l'une des revendications 1 à 10, dans lequel le système de circulation d'air comporte des moyens de chauffage du flux d'air.

12. Dispositif selon l'une des revendications 1 à 11, dans lequel une rampe d'aspersion d'eau fait saillie à l'intérieur de la cage de façon à asperger la face interne exposée des échantillons.

13. Procédé de test de vieillissement artificiel accéléré d'échantillons, dans lequel on expose des échantillons à des rayonnements photoniques et à des agents climatiques, **caractérisé en ce qu'**il met en oeuvre des étapes consistant à
- disposer les échantillons (E, E1, E2, ..., E9) sur une cage (40) porte-échantillons rotative autour d'un axe sensiblement horizontal (H-H),
- positionner fixement (15), et alimenter électriquement, au moins une lampe (10) à rayonnement photonique, dans la partie centrale (45) de la cage, selon l'axe de rotation horizontal (H-H),
- générer un flux d'air direct simple arrivant directement sur la lampe (10) en se propageant parallèlement sensiblement à l'axe, le flux d'air divergeant autour de la lampe et s'évacuant sensiblement perpendiculairement à l'axe horizontal (H-H) à la périphérie (41) de la cage porte-échantillons, si bien que le flux d'air s'échauffe au contact de la lampe, avant d'envelopper les échantillons, et
- baigner une portion inférieure de la cage porte-échantillons rotative dans un bain liquide, de façon à alterner cycliquement des phases d'immersion des échantillons dans le liquide avec des phases d'émersion des échantillons dans le flux d'air.

14. Procédé de test selon la revendication 13, dans lequel on baigne la portion inférieure de la cage dans une cuve apte à contenir un volume de liquide.

15. Procédé de test selon l'une des revendications 13 à 14, dans lequel le flux d'air est un flux tourbillonnaire autour de l'axe de rotation sensiblement horizontal (H-H), le flux d'air ayant, dans la partie centrale (45,46) de la cage un mouvement de translation dont la composante est sensiblement parallèle à la direction axiale (H-H), le flux d'air ayant, au niveau de la périphérie (41) de la cage, un mouvement de rotation dont chaque composante est comprise entre la direction tangentielle et la direction radiale.

16. Procédé de test selon l'une des revendications 13 à 15, comportant un protocole d'essais consistant à :
- mesurer et réguler la température d'un élément de référence par une régulation de température agissant sur l'alimentation électrique (K1,K2) de la lampe (10) et/ou sur les moyens (18,19) de génération du flux d'air.

17. Procédé de test selon l'une des revendications 13 à 16, comportant un protocole d'essais avec des phases d'immersion dans lesquelles le liquide baignant la cage porte-échantillons est de l'eau contenant de l'oxygène dissous.

18. Procédé de test selon l'une des revendications 13 à 17 **caractérisé en ce que** le liquide est de l'eau additionnée d'un ou de polluants choisis parmi le groupe de polluants comportant l'ozone, l'anhydride carbonique, les oxydes d'azote, les anhydrides sulfuriques et sulfureux, les chlorures, les composés de fluor, les composés de phosphore, les insecticides, les pesticides et les aérosols.

## Claims

1. Device for exposure of samples to photonic radiation for accelerated artificial weathering tests of samples, comprising:
- a chamber (30),
- a sample-holding cage (40) rotating around an axis (H-H) adapted to be approximately horizontal in operation,
- means (15) of fixedly positioning near the central part of the cage (45, 46) and of supplying with electricity at least one low power arc lamp (10) with a power of the order of a few tens to about a thousand watts, and particularly a mercury vapour lamp or a mercury compound vapour lamp, and
- an air circulation system comprising an air passage orifice (50) arranged along the axial prolongation (H-H) of the central part of the cage (45, 46) and several fixed air passage openings (31 - 38) arranged in the radial prolongation of the periphery of the cage (40), the air circulation being designed to keep the samples in the chamber at a uniform temperature of a few tens of degrees Celsius,
- **characterized in that** ventilation means (1, 18, 19) blow air into the chamber (30) through the orifice (50) that forms an air intake orifice oriented approximately parallel to the horizontal axis (H-H), the lamp (10) being placed along the immediate axial prolongation (H-H) of the air intake orifice (50), so as to generate an air stream arriving directly on the lamp (10) and approximately parallel to the axis in the central part of the cage (45, 46), the air stream diverging around the lamp and being evacuated approximately perpendicular to the axis through the fixed openings (31 - 38) that form air outlet openings at the periphery of the cage (40), such that the temperature of the air stream increases as it comes into contact with the lamp, before going around the samples, thereby increasing and then keeping uniform their temperature.

2. Device according to claim 1, in which the air stream diverging around the lamp comprises essentially a component approximately parallel to the axis (H-H), in the central part of the cage (45 - 46) close to the lamp (10), while the said air stream comprises essentially components approximately perpendicular to the axis, at the periphery of the cage (41) near the samples.

3. Device according to either of claims 1 and 2, in which the assembly of the lamp positioning means (15) and power supply means (10), the ventilation and air circulation means (1, 18, 19) and the cage rotation drive means (50) are arranged in line along the horizontal axis (H-H) of the device.

4. Device according to one of claims 1 to 3, in which the cage (40) is rotatingly installed on a hub (50) hollowed out along the axial direction (H-H), the air stream being blown through the axial hole through the hub.

5. Device according to claim 4, in which the air circulation system comprises ventilation means (1, 18, 19) arranged in the hollowed out hole in the hub, so as to blow air towards the chamber (30).

6. Device according to one of claims 1 to 5, in which the air circulation system comprises a series of several fixed air outlet openings (31 - 38) arranged regularly at the periphery (41) of the cage (40) with a symmetry of revolution about the axis (H-H), each fixed opening (31, 32, 33, ..., 38) being oriented in a direction between the radial direction and the tangential direction at the periphery (41) of the cage.

7. Device according to one of claims 1 to 6, in which the cylindrical chamber (30) comprises air outlet openings (31 - 38) arranged approximately at the periphery (41) of the cage, each air outlet opening (31) being oriented in a non-radial direction and with a depth, aperture and angle of inclination relative to the radial direction, such that the radiation from each lamp (10) positioned in the central part of the cage (40) preferably does not pass through the opening (31) and preferably does not escape directly outside the chamber (30).

8. Device according to one of claims 4 to 7, in which the cage (40) is connected to the hub (50) through a declutchable drive mechanism (51, 55, 56, 57) allowing free rotation of the cage.

9. Device according to claim 8, in which the mechanism (50, 51) comprises rotation declutching means (55, 56, 57) under the effect of a limit axial torque between the cage (40) and the hub (50).

10. Device according to one of claims 1 to 9, comprising a tank (6) arranged in the lower part of the device and intended to contain a liquid volume so that a lower portion of the rotating sample-holding cage can be immersed, such that the samples cyclically alternate between immersion phases in the liquid and emersion phases in the air stream.

11. Device according to claims 1 to 10, in which the air circulation system comprises means of heating the air stream.

12. Device according to one of claims 1 to 11, in which a water sprinkling header pipe projects inside the cage so as to sprinkle the exposed inner face of the samples.

13. Method for carrying out accelerated artificial weathering tests on samples, in which samples are exposed to photonic radiation and climatic agents, **characterised in that** it includes steps consisting of:
- arranging the samples (E, E1, E2, ..., E9) on a sample-holding cage (40) rotating about an approximately horizontal axis (H-H),
- fixingly positioning (15) and supplying electrical power to at least one photonic radiation lamp (10) in the central part (45) of the cage along the horizontal rotation axis (H-H),
- generating a simple direct air stream arriving directly on the lamp (10) while propagating approximately parallel to the axis, the air stream diverging around the lamp and being evacuated approximately perpendicular to the horizontal axis (H-H) at the periphery (41) of the sample-holding cage, such that the air stream temperature increases on contact with the lamp, before surrounding the samples, and,
- immersing a lower portion of the rotating sample-holding cage in a liquid bath, so as to cyclically alternate phases in which the samples are immersed in the liquid, with phases in which the samples are exposed to the air stream.

14. Test method according to claim 13, in which the lower portion of the cage is immersed in a tank that can contain a liquid volume.

15. Test method according to one of claims 13 to 14, in which the air stream is a stream swirling about the approximately horizontal axis of rotation (H-H), the air stream having in the central part (45, 46) of the cage a translation movement whose component is approximately parallel to the axial direction (H-H), the air stream having at the level of the periphery (41) of the cage, a rotation movement of which each component is in a direction between the radial direction and the tangential direction.

16. Test method according to one of claims 13 to 15, comprising a test protocol consisting of:
- measuring and regulating the temperature of a reference element by a temperature regulation acting on the electrical power supply (K1, K2) to the lamp (10) and / or the air stream generation means (18, 19).

17. Test method according to one of claims 13 to 16, comprising a test protocol with immersion phases in which the liquid in which the sample-holding cage is immersed is water containing dissolved oxygen.

18. Test method according to one of claims 13 to 17, **characterised in that** the liquid is water containing one or more pollutants chosen from among the group of pollutants comprising ozone, carbon dioxide, nitrogen oxides, sulphur trioxide and sulphur dioxide, chlorides, fluorine compounds, phosphorus compounds, insecticides, pesticides and aerosols.

## Patentansprüche

1. Vorrichtung zur Bestrahlung mit Photonen für Versuche zur beschleunigten künstlichen Alterung von Proben, umfassend:
- eine Umhüllung (30),
- einen Probenträgerkäfig (40), der um eine Achse (H-H) rotationsfähig ist, die dazu bestimmt ist, während des Betriebs im Wesentlichen horizontal zu sein,
- Mittel (15), um mindestens eine Lichtbogenlampe (10) mit geringer Leistung in der Größenordnung von mehreren Dutzend bis etwa eintausend Watt, insbesondere eine Quecksilberdampf- oder Quecksilberverbindungsdampflampe, fest in dem mittleren Abschnitt des Käfigs (45, 46) zu positionieren und mit Strom zu versorgen,
- ein Luftzirkulationssystem, das eine Luftdurchgangsöffnung (50), die in der axialen Verlängerung (H-H) des mittleren Abschnitts des Käfigs (45, 46) angeordnet ist, und mehrere ständige Luftdurchgangsöffnungen (31-38), die in der radialen Verlängerung des Umfangs des Käfigs (40) angeordnet sind, aufweist, wobei die Luftzirkulation dazu bestimmt ist, die Proben in der Umhüllung auf einer homogenen Temperatur zu halten, die mehrere Dutzend Grad Celsius beträgt,
- **dadurch gekennzeichnet, dass** Belüftungsmittel (1, 18, 19) Luft in die Umhüllung (30) durch die Öffnung (50) hindurch einblasen, welche eine Lufteintrittsöffnung bildet, die im Wesentlichen parallel zur horizontalen Achse (H-H) ausgerichtet ist, wobei die Lampe (10) in der unmittelbaren axialen Verlängerung (H-H) der Lufteintrittsöffnung (50) angeordnet ist, derart, dass ein Luftstrom erzeugt wird, der direkt auf die Lampe (10) auftrifft und im mittleren Abschnitt des Käfigs (45, 46) im Wesentlichen parallel zur Achse ist, wobei der Luftstrom um die Lampe herum divergiert und im Wesentlichen senkrecht zur Achse durch die ständigen Öffnungen (31-38), welche Luftaustrittsöffnungen bilden, am Umfang des Käfigs (40) austritt, so dass sich der Luftstrom beim Kontakt mit der Lampe erwärmt, bevor er die Proben umhüllt und dabei deren Temperatur erhöht und dann reguliert.

2. Vorrichtung nach Anspruch 1, wobei der um die Lampe herum divergierende Luftstrom im Wesentlichen eine Komponente aufweist, die im mittleren Abschnitt des Käfigs (45-46), in der Nähe der Lampe (10), im Wesentlichen parallel zur Achse (H-H) ist, während der Luftstrom am Umfang des Käfigs (41) im Bereich der Proben im Wesentlichen Komponenten aufweist, die im Wesentlichen senkrecht zur Achse sind.

3. Vorrichtung nach einem der Ansprüche 1 bis 2, wobei die Gesamtheit der Mittel (15) zur Positionierung und Stromversorgung der Lampe (10), der Mittel zur Belüftung und Luftzirkulation (1, 18, 19) und der Mittel zum Drehantrieb des Käfigs (50) in einer Linie entlang der horizontalen Achse (H-H) der Vorrichtung angeordnet ist.

4. Vorrichtung nach einem der Ansprüche 1 bis 3, wobei der Käfig (40) drehbar auf einer axial (H-H) ausgesparten Nabe (50) angebracht ist, wobei der Luftstrom in die axiale Aussparung der Nabe eingeblasen wird.

5. Vorrichtung nach Anspruch 4, wobei das Luftzirkulationssystem Belüftungsmittel (1, 18, 19) aufweist, die in der Aussparungsöffnung der Nabe angeordnet sind, derart, dass die Luft in Richtung der Umhüllung (30) eingeblasen wird.

6. Vorrichtung nach einem der Ansprüche 1 bis 5, wobei das Luftzirkulationssystem eine Reihe von mehreren ständigen Luftaustrittsöffnungen (31-38) aufweist, die gleichmäßig auf dem Umfang (41) des Käfigs (40) mit einer Rotationssymmetrie um die Achse (H-H) angeordnet sind, wobei jede ständige Öffnung (31, 32, 33, ..., 38) in einer Richtung ausgerichtet ist, die zwischen der radialen Richtung und der zum Umfang des Käfigs (41) tangentialen Richtung liegt.

7. Vorrichtung nach einem der Ansprüche 1 bis 6, wobei die zylindrische Umhüllung (30) Luftaustrittsschlitze (31-38) aufweist, die im Wesentlichen am Umfang des Käfigs (41) angeordnet sind, wobei jeder Luftaustrittsschlitz (31) nichtradial ausgerichtet ist und eine Tiefe, eine Öffnungsweite und einen Neigungswinkel bezüglich der radialen Richtung aufweist, derart, dass die Strahlung jeder Lampe (10), die im mittleren Abschnitt des Käfigs (40) positioniert ist, vorzugsweise nicht den Schlitz (31) durchquert und vorzugsweise nicht direkt aus der Umhüllung (30) entweicht.

8. Vorrichtung nach einem der Ansprüche 4 bis 7, wobei der Käfig (40) mit der Nabe (50) durch einen auskuppelbaren Antriebsmechanismus (51, 55, 56, 57) verbunden ist, der eine freie Drehung des Käfigs ermöglicht.

9. Vorrichtung nach Anspruch 8, wobei der Mechanismus (50, 51) Mittel zum Auskuppeln bei einer Rotation (55, 56, 57) unter der Einwirkung eines axialen Grenzmoments zwischen dem Käfig (40) und der Nabe (50) aufweist.

10. Vorrichtung nach einem der Ansprüche 1 bis 9, der eine Wanne (6) aufweist, die im unteren Teil der Vorrichtung angeordnet ist und dazu bestimmt ist, eine Flüssigkeitsmenge zu enthalten, so dass ein unterer Abschnitt des rotationsfähigen Probenträgerkäfigs eingetaucht wird, derart, dass die Proben zyklisch abwechselnd Phasen des Eintauchens in die Flüssigkeit und Phasen des Austritts in den Luftstrom durchlaufen.

11. Vorrichtung nach einem der Ansprüche 1 bis 10, wobei das Luftzirkulationssystem Mittel zur Erwärmung des Luftstroms aufweist.

12. Vorrichtung nach einem der Ansprüche 1 bis 11, wobei eine Rampe zum Besprühen mit Wasser in das Innere des Käfigs hineinragt, so dass die exponierte Innenseite der Proben besprüht wird.

13. Testverfahren zur beschleunigten künstlichen Alterung von Proben, bei welchem die Proben Photonenstrahlungen und klimatischen Einwirkungen ausgesetzt werden, **dadurch gekennzeichnet, dass** die folgenden Schritte ausgeführt werden:
- Anordnen der Proben (E, E1, E2, ..., E9) auf einem Probenträgerkäfig (40), der um eine Achse (H-H) rotationsfähig ist, die im Wesentlichen horizontal ist;
- festes Positionieren (15) mindestens einer Lampe (10) zur Photonenbestrahlung im mittleren Abschnitt (45) des Käfigs entlang der horizontalen Rotationsachse (H-H) und Versorgen derselben mit Strom;
- Erzeugen eines einfachen direkten Luftstroms, der direkt auf die Lampe (10) auftrifft, indem er sich im Wesentlichen parallel zur Achse ausbreitet, wobei der Luftstrom um die Lampe herum divergiert und im Wesentlichen senkrecht zur horizontalen Achse (H-H) am Umfang (41) des Probenträgerkäfigs austritt, so dass sich der Luftstrom beim Kontakt mit der Lampe erwärmt, bevor er die Proben umgibt, und
- Eintauchen eines unteren Abschnitts des rotationsfähigen Probenträgerkäfigs in ein Flüssigkeitsbad, so dass sich Phasen des Eintauchens der Proben in die Flüssigkeit mit Phasen des Austritts der Proben in den Luftstrom zyklisch abwechseln.

14. Testverfahren nach Anspruch 13, wobei der untere Abschnitt des Käfigs in eine Wanne eingetaucht wird, die geeignet ist, eine Flüssigkeitsmenge zu enthalten.

15. Testverfahren nach einem der Ansprüche 13 bis 14, wobei der Luftstrom ein Wirbelstrom um die im Wesentlichen horizontale Rotationsachse (H-H) herum ist, wobei der Luftstrom im mittleren Abschnitt (45, 46) des Käfigs eine Translationsbewegung aufweist, deren Komponente im Wesentlichen parallel zur axialen Richtung (H-H) ist, wobei der Luftstrom im Bereich des Umfangs (41) des Käfigs eine Rotationsbewegung aufweist, deren jede Komponente zwischen der tangentialen Richtung und der radialen Richtung verläuft.

16. Testverfahren nach einem der Ansprüche 13 bis 15, welches ein Versuchsprotokoll aufweist, das in Folgendem besteht:
- Messen und Regeln der Temperatur eines Bezugselements durch eine Temperaturregelung durch Einwirkung auf die Stromversorgung (K1, K2) der Lampe (10) und/oder auf die Mittel (18, 19) zur Erzeugung des Luftstroms.

17. Testverfahren nach einem der Ansprüche 13 bis 16, welches ein Versuchsprotokoll mit Phasen des Eintauchens aufweist, bei welchen die Flüssigkeit, in die der Probenträgerkäfig eingetaucht wird, Wasser ist, das gelösten Sauerstoff enthält.

18. Testverfahren nach einem der Ansprüche 13 bis 17, **dadurch gekennzeichnet, dass** die Flüssigkeit Wasser ist, dem ein Verunreinigungsstoff zugesetzt ist oder dem Verunreinigungsstoffe zugesetzt sind, die aus der Gruppe von Verunreinigungsstoffen gewählt sind, welche Ozon, Kohlendioxid, Stickoxide, Schwefeltrioxid und Schwefeldioxid, Chloride, Fluorverbindungen, Phosphorverbindungen, Insektizide, Pestizide und Aerosole umfasst.
